(19) Europäisches Patentamt
European Patent Office
Office européen des brevets

(11) **EP 2 711 688 B2**

(12) **NEW EUROPEAN PATENT SPECIFICATION**
After opposition procedure

(45) Date of publication and mention
of the opposition decision:
**15.07.2026   Bulletin 2026/29**

(45) Mention of the grant of the patent:
**02.09.2020   Bulletin 2020/36**

(21) Application number: **12789485.5**

(22) Date of filing: **17.05.2012**

(51) International Patent Classification (IPC):
**G01N 21/3504** (2014.01)    **G01N 21/15** (2006.01)
**G01N 21/85** (2006.01)    G01N 21/17 (2006.01)

(52) Cooperative Patent Classification (CPC):
**G01N 21/3504; G01N 21/15; G01N 21/8507;**
G01N 2021/151; G01N 2021/1712

(86) International application number:
**PCT/JP2012/062595**

(87) International publication number:
**WO 2012/161067 (29.11.2012 Gazette 2012/48)**

(54) **MEASURING UNIT AND GAS ANALYZING APPARATUS**

MESSEINHEIT UND VORRICHTUNG ZUR GASANALYSE

UNITÉ DE MESURE ET DISPOSITIF D'ANALYSE DE GAZ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.05.2011   JP 2011113789**

(43) Date of publication of application:
**26.03.2014   Bulletin 2014/13**

(73) Proprietor: **HORIBA, Ltd.**
**Minami-ku
Kyoto-shi
Kyoto 601-8510 (JP)**

(72) Inventors:
• **UKON, Juichiro**
**Kyoto-shi, Kyoto 601-8510 (JP)**
• **IDO, Takuya**
**Kyoto-shi, Kyoto 601-8510 (JP)**
• **OHNISHI, Toshikazu**
**Kyoto-shi, Kyoto 601-8510 (JP)**
• **TSUJIMOTO, Toshiyuki**
**Kyoto-shi, Kyoto 601-8510 (JP)**

(74) Representative: **Müller Hoffmann & Partner**
**Patentanwälte mbB
St.-Martin-Straße 58
81541 München (DE)**

(56) References cited:
WO-A1-2006/030059        JP-A- 2001 021 493
JP-A- 2001 059 829        JP-A- 2001 523 818
JP-A- 2006 227 003        JP-A- 2011 033 351

US-A- 4 126 396        US-A- 4 310 762
US-A- 4 549 080        US-A- 4 560 873
US-A- 5 689 114        US-A- 5 984 998
US-A- 6 044 329        US-A- 6 150 661
US-A1- 2006 176 486    US-B1- 6 483 589

• offenkundige Vorbenutzung GM700, TDLS-Analysator für NH3,HF, HCl oder 02 der Firma SICK MAIHAK GmbH (heute SICK AG) mit den Dokumenten
• Betriebsanleitung GM700, TDLS-Analysator für NH3, HF, HCl oder 02, Ausführung mit Messlanze, vom Februar 2007, Materialnummer 8010099
• Rechnung und Lieferschein eines GM700, Ausführung mit Messlanze mit der Materialnummer 1023006 (GMM700-0511 Sende/Empfangseinheit) bzw. der Materialnummer 1043716 (GMP-700-1242 Messlanze) vom 29.05.2008
• Entwicklungsdatenbank (EDB) - Auszüge zur Sende/Empfangseinheit mit der Materialnummer 1023006 und Messlanze mit der Materialnummer 1043716
• Optikschema NH3, Zeichnungsnummer 9070314, enthalten in Dokumentstückliste 9070151 00
• Datenblatt Photodiodes EPITAXX ETX1000T
• Lanze, GMP, Zeichnungsnummer 9090107, enthalten in Stückliste zur Messlanze mit der Materialnummer 1043716
• Kopie der Frachtpapiere der Lieferung des in der E1 b in Rechnung gestellten und spezifizierten GM700 Gasanalysators

- **Bestätigung der Firma TITAN CEMENT SA über den Empfang des in der E1b in Rechnung gestellten und spezifizierten GM70O Gasanalysators**
- **Radiation damage studies of optoelectronic components for the CMS tracker optical links RADECS 97. Fourth European Conference on Radiation and its Effects on Components and Systems (Cat NO.97TH8294), October 1997**

- **A solid-state hyperspectral imager for real-time standoff explosives detection using shortwave infrared imaging - Proceedings of SPIE - The International Society for Optical Engineering 7310. May 2009**

## Description

### TECHNICAL FIELD

The present invention relates to a gas analyzing apparatus and a measurement unit. More specifically, the present invention relates to a gas analyzing apparatus that analyzes a concentration of a predetermined component in a sample gas using a light absorption technique, and a measurement unit used in the gas analyzing apparatus.

### BACKGROUND ART

A combustion exhaust gas, which is expelled from a boiler that combusts coal or heavy oil, includes gases such as NOx, SOx, CO2, CO, etc. Gas analyzing apparatuses for analyzing contents of these components in the gas have previously been developed. Various types of apparatuses have been developed, such as an open-path type apparatus and a probe-type apparatus.

One example of a cylindrical measurement unit used in the above-described probe-type gas analyzing apparatus is disclosed in US 6809825. The measurement unit disclosed therein emits a measurement light from a light source that is arranged at one end side of a cylindrical casing so as to pass the measurement light through a sample gas that is introduced into the inner space of the casing. The measurement light is reflected by a reflecting mirror that is arranged at another end side of the casing and the reflected measurement light is received by a light receiving sensor. An amount of the measurement light absorbed by the sample gas is derived by subtracting one information of the measurement light from another. One information is the information that can be derived from the light receiving sensor. Another is the information of the measurement light at the time when the measurement light is emitted from the light source. Then, the concentration of the predetermined component in the sample gas can be derived based on the amount of the absorption of the measurement light.

Due to such a measurement principle, in order to perform accurate analyses in the gas analyzing apparatus using the above-described measurement light, it is important to receive the measurement light within the light receiving plane of the light receiving sensor. From such a point of view, it is considered that the positioning of the optical components, such as the light source, the reflecting mirror, the light receiving, etc., can be performed in the probe-type gas analyzing apparatuses easier than the open-path type gas analyzing apparatus. This is because the optical components of the probe-type gas analyzing apparatus are fixed in a single casing as described above. On the other hand, these optical components of the open-path type gas analyzing apparatus are arranged separately. In other words, it is considered to be easier to set the irradiation point of the measurement light within the light receiving plane of the light receiving sensor in the probe-type gas analyzing apparatus.

US 6,044,329 and US 4,549,080 disclose gas analyzers where purge gas is introduced into a space separated from a space where sample gas flows.

US 2006/176486 discloses to introduce purge gas directly into a funnel with sample gas.

US 5,984,998 discloses the introduction of purge gas without mixing it with sample gas US 4,560,873 discloses a combustion gas analyzer comprising a transceiver and a probe with a measurement cavity through which the stack gases are passed. The analyzer furthermore comprises purge lines for purging the transceiver and the probe.

US 4,126,396 discloses a device for analyzing stack gases comprising pipes for scavenging air for preventing impurities becoming deposited on optical components.

### DISCLOSURE OF INVENTION

### TECHNICAL PROBLEM

However, even with the above-described probe-type gas analyzing apparatus, situations arise in which the irradiation point of the measurement light cannot be set within the light receiving plane of the light receiving sensor.

In measurement units, in which the optical components such as the above-described sensor, the reflecting mirror, etc. are used, there is the case in which a cleaning air (so-called purge air) is introduced around the optical components in the casing. The cleaning air is introduced with a predetermined pressure in order to avoid the contamination of the optical components due to dust contained in the sample gas, etc.

While the temperature of the sample gas expelled from the above-described boiler is very high, the temperature of the purge air is typically the same as the ambient temperature. When there is a temperature difference between the purge air and the sample gas, spatial distribution of temperature occurs inside the casing of the measurement unit, such as on the path of the measurement light. When such a spatial distribution of temperature occurs, the spatial refractive index is changed proportionally to the spatial distribution of temperature. Then, the measurement light propagating in the space might be refracted since the change of the refractive index causes the effect equivalent to transitional optical lenses (so-called thermal lens effect).

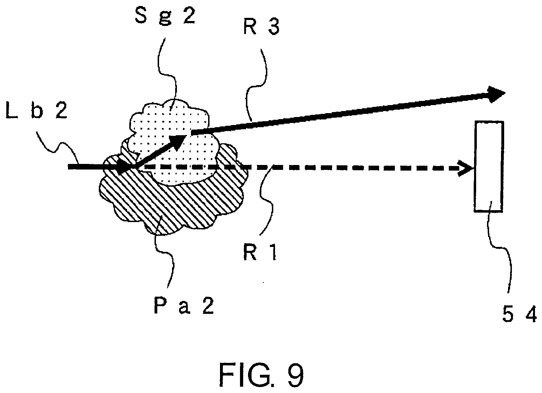

As shown in Fig. 9, the measurement light Lb2 should propagate on a straight path R1. However, Lb2 might propagate on a refracted path, like the path R3, due to the temperature difference between the sample gas Sg2 and the purge air Pa2.

Fig. 9 is an image view showing that the measurement light is improperly received in the conventional measurement unit. If the measurement light Lb2 is refracted in such a manner, the measurement light Lb2 cannot be received within the light receiving plane of the light receiving sensor. Therefore, it is sometimes difficult to perform an accurate analysis.

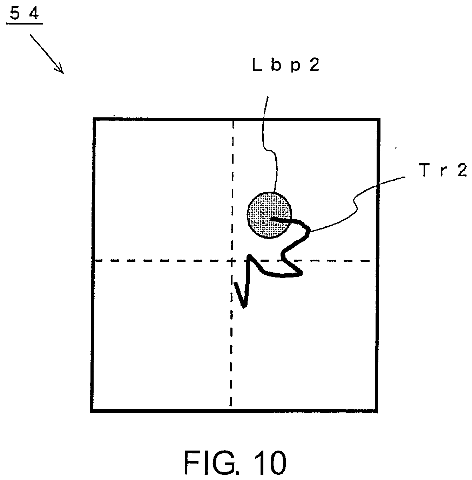

In addition, the state of the refraction of the measurement light Lb2 changes with time. This is due to the thermal lens effect changing because of changes in the flows of the sample gas Sg2 and the purge air Pa2. As a result, even if the measurement light Lb2 is emitted onto the light receiving plane of the light receiving sensor 54, the irradiation point Lbp2 of the measurement light Lb2 on the light receiving plane may fluctuate, as shown in Fig. 10.

Fig. 10 is a view showing that the irradiation point Lbp2 fluctuates on the light receiving plane in the conventional measurement unit. In Fig. 10, a locus line Tr2 is a movement locus of the irradiation point Lbp2. In Fig. 10, since the locus line Tr2 snakes, it is shown that the irradiation point Lbp2 fluctuates as described above. When the position of the irradiation point Lbp2 moves in the light receiving sensor, stable signals might not be derived from the light receiving sensor, even if the intensity of the measurement light Lb2 is constant, because the light receiving sensitivity of the light receiving sensor may be dependent on the positions of the light receiving plane.

The measurement light might also be refracted, due to the thermal lens effect, in the open-path gas analyzing apparatus when using the purge gas in the same manner as the probe-type gas analyzing apparatus. In such a configuration, the irradiation point of the measurement light sometimes cannot be set properly within the light receiving plane of the light receiving sensor.

The present invention was conceived in light of the above-described problems and the object of the present invention is to provide the measurement unit and the gas analyzing apparatus that can analyze the sample gas more accurately than the conventional techniques.

TECHNICAL SOLUTION

The above objects are solved by the claimed matter according to the independent claim.

A measurement unit, according to one aspect of the present invention, is the measurement unit that is used in an analyzing apparatus for measuring concentrations of component gases in a sample gas. The measurement unit comprises a light emitting unit, a light receiving unit, a purge air introducing unit, and a condensing lens. The light emitting unit is configured to emit a measurement light to the sample gas. The light receiving unit is configured to receive the measurement light on a light receiving plane. The purge air introducing unit is configured to introduce a purge air into a vicinity of at least one of the light emitting unit and the light receiving unit. The condensing lens is arranged in an optical path of the measurement light. The optical path of the measurement light extends from the light emitting unit to the light receiving unit. The condensing lens is configured to condense, within the light receiving plane of the light receiving unit, the measurement light. In this case, the propagation path of the measurement light is variable due to a thermal lens effect caused by a temperature difference between the sample gas and the purge air.

The measurement light can be properly received within the light receiving plane of the light receiving unit, even if the path of the measurement light is refracted due to the thermal lens effect. In addition, the measurement light can be stably emitted to the predetermined position of the light receiving plane. Therefore, the information of the measurement light can be accurately derived with the light receiving unit, and accurate analysis of the predetermined component gas, in the sample gas, can be performed based on such information.

The condensing lens may be arranged immediately in front of the light receiving unit. The measurement unit may further include an optical window arranged immediately in front of the condensing lens. The optical window is configured to protect at least the condensing lens. The purge air introducing unit may introduce the purge air immediately in front of the optical window.

Optical components can be properly protected by introducing the purge air in the appropriate position. Even if the thermal lens effect occurs when the purge air is introduced, the measurement light can properly be received by the light receiving plane of the light receiving unit by utilizing the condensing lens.

The measurement unit further comprises a cylindrical probe tube having openings to introduce the sample gas into the probe tube. The purge air introducing unit may introduce the purge air inside the probe tube. The light emitting unit may emit the measurement light to the sample gas introduced inside the probe tube.

By utilizing the condensing lens, the measurement light can properly be received within the light receiving plane of the light receiving unit. This is especially true for a probe-type measurement unit, in which bending of the measurement light due to the thermal lens effect easily occurs.

The measurement unit may further comprise a reflection mirror arranged at one end portion of the probe tube. The light emitting unit may be arranged at another end portion of the probe tube. The light emitting unit is configured to emit the measurement light toward the reflection mirror. The light receiving unit may be arranged at the another end portion of the probe tube. The light receiving unit is configured to receive the measurement light that has been reflected by the reflection mirror.

The numerical aperture of the condensing lens is greater than or equal to 0.08.

With this configuration, when the light receiving plane of the light receiving unit is formed with a multi-layered semiconductor, the interference of the measurement light can be inhibited. The interference is caused by the multiple reflections of the measurement light that occur at the interfaces of the semiconductor layers. The intensity of the measurement light can be detected accurately with the light receiving unit.

The light receiving unit is tilted with respect to the condensing lens so that an angle between the light receiving plane and an image formation plane of the condensing lens is greater than or equal to 10 degrees.

With this configuration, when the light receiving plane of the light receiving unit is formed with a multi-layered semiconductor, the interference of the measurement light can be inhibited. The interference is caused by the multiple reflections of the measurement light that occur at the interfaces of the semiconductor layers. The intensity of the measurement light can be detected accurately with the light receiving unit.

BRIEF DESCRIPTION OF DRAWINGS

Fig. 1 is an external view of the measurement unit 1 according to the first embodiment.
Fig. 2 is a cross-sectional view showing the inner structure of the measurement unit 1 according to the first embodiment.
Fig. 3 is an image view showing that the measurement light Lb1 refracted in the measurement unit 1 is guided within the light receiving plane of the light receiving unit 24 by the condensing lens 23.
Fig. 4 is a view showing that the movement of the irradiation point Lbp1 on the light receiving plane is inhibited in the measurement unit 1.
Fig. 5 is a cross-sectional view showing the detailed structure of the light receiving unit 24.
Fig. 6 is a graph showing the stabilities of the electrical signals of the light receiving unit 24 derived at each of settings of the condensing lens 23 and the light receiving unit 24 in the measurement unit 1 according to the first embodiment.
Fig. 7 is an enlarged view of a part of Fig. 6.
Fig. 8 is a cross-sectional view showing the inner structure of the measurement unit 2 according to the second example, which does not form part of the invention.
Fig. 9 is an image view showing that the measurement light Lb2 is not received properly in the conventional measurement unit.
Fig. 10 is a view showing that the irradiation point Lbp2 fluctuates on the light receiving plane in the conventional measurement unit.

EMBODIMENT

(First Embodiment)

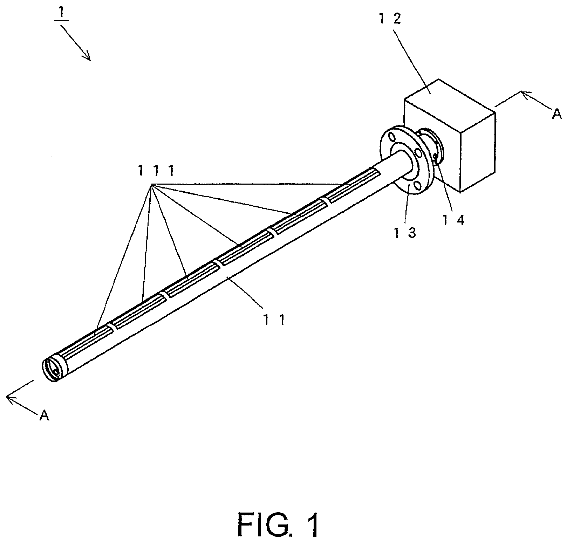

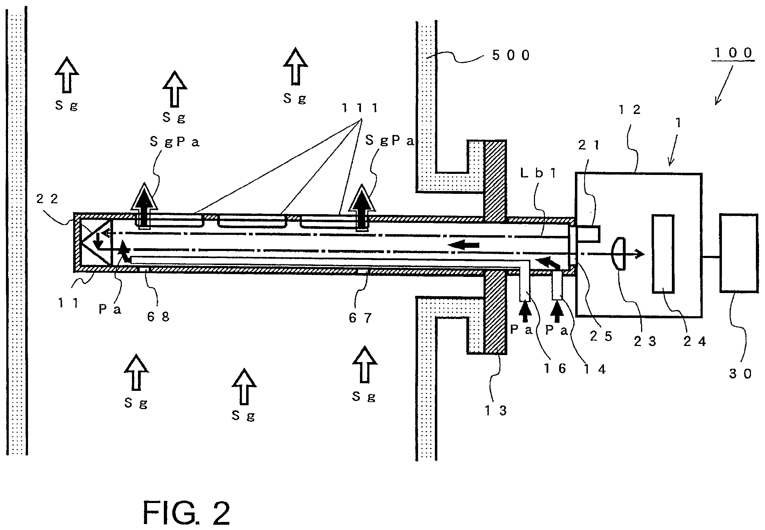

A measurement unit 1 and a gas analyzing apparatus 100 using the measurement unit 1 will be explained below. The gas analyzing apparatus 100 is a so-called probe-type gas analyzing system and the measurement unit 1 is a so-called probe unit. First, the structure of the measurement unit 1 will be explained, referring to Fig. 1 and Fig. 2. Fig. 1 is an external view of the measurement unit 1 according to the first embodiment. Fig. 2 is a cross-sectional view showing the inner structure of the measurement unit 1 according to the first embodiment. Fig. 2 is a view that includes the A-A cross section of the measurement unit 1 shown in Fig. 1. As shown in Fig. 1, the measurement unit 1 includes a probe tube 11, an optical unit 12, and a flange 13.

The probe tube 11 is a cylindrical member in which introducing openings 111 are formed. The introducing openings 111 introduce a sample gas Sg inside the probe tube 11 by diffusion of the sample gas Sg. The probe tube 11 may be made of any metallic material appropriate for the environment where the measurement unit 1 is used. As shown in Fig. 1, the introducing openings 111 are formed as intermittent slits on the side plane of the probe tube 11. As shown in Fig. 2, a reflection mirror 22 is arranged at one inner end side portion of the probe tube 11. On the other hand, the other end side portion of the probe tube 11 is connected to the optical unit 12.

As shown in Fig. 2, the optical unit 12 is the optical apparatus that includes a light emitting unit 21, a condensing lens 23, a light receiving unit 24, and an optical window 25. The light emitting unit 21 is the light source apparatus that emits a measurement light Lb1 to the inside of the probe tube 11. The light emitting unit 21 is typically a light source apparatus that emits the light with a predetermined wavelength band, such as an infrared laser oscillating apparatus, an LED (Light Emitting Diode), or a deuterium lamp that emits an ultraviolet light. The light receiving unit 24 is the light receiving apparatus that receives the measurement light Lb1 on the light receiving plane. The light receiving unit 24 is typically a photoelectric converting apparatus, such as a photodiode. The condensing lens 23 is the lens member that condenses the measurement light Lb1 within the light receiving plane of the light receiving unit 24. The condensing lens 23 is arranged immediately in front of the light receiving unit 24. The light receiving unit 24 is electrically connected to a processing apparatus 30, and sends information (for example, an intensity) of the measurement light Lb1 to the processing apparatus 30 as an electric signal. The optical window 25 is the planar member that is made of the material that transmits the measurement light Lb1. As shown in Fig. 2, the optical window 25 may be arranged at the point where the casing of the optical unit 12 and the probe tube 11 are connected. In other words, the optical window 25 may be disposed immediately in front of the light emitting unit 21 and the condensing lens 23. The optical window 25 protects the light emitting unit 21, and the condensing lens 23. It should be noted that the above-described reflection mirror 22 is arranged inside the probe tube 11 in advance, so as to reflect the measurement light Lb1. Measurement light Lb1 is emitted from the light emitting unit 21, toward the light receiving unit 24.

The processing apparatus 30 controls the operations of the light emitting unit 21 and the light receiving unit 24 and calculates the concentration of the predetermined component of the sample gas Sg in the probe tube 11 based on the signal received from the light receiving unit 24. The processing apparatus 30 typically includes an information processing apparatus, such as a CPU (Central Processing Unit), etc., a storing apparatus, such as a memory, etc., an interface apparatus that receives the operations from a user, a displaying apparatus that displays results of the analysis, etc. The processing apparatus 30 performs the arithmetic processes based on the operations by the user and the program stored in the storing apparatus.

As shown in Fig. 2, in the above-described probe tube 11, a purge air introducing port 14 is arranged inside the probe tube 11, the purge air introducing port 14 introduces a purge air Pa. The purge air introducing port 14 is arranged in the vicinity of the connection part at which the probe tube 11 and the optical unit 12 are connected, as shown in Fig. 1 and Fig. 2. Arranged in such a manner, introducing the purge air Pa with the predetermined pressure from the purge air introducing port 14 prevents the sample gas Sg and dust inside the probe tube 11 from touching the optical window 25 of the optical unit 12. Therefore, the contamination and corrosion of the optical window 25 can be inhibited. The flow paths of the purge air Pa are shown in thick black arrows in Fig. 2. In addition, the flow paths of the sample gas Sg is shown in white arrows in Fig. 2. It is preferable that the above-described purge air introducing port 14 is arranged so as to introduce the purge air immediately in front of the optical window 25. The optical components such as the condensing lens 23, etc. can be properly protected by introducing the purge air Pa at such an appropriate position.

The probe tube 11 further includes a purge air introducing pipe 16 that introduces the purge air Pa in front of the reflection mirror 22 to protect the reflection mirror 22. Such a structure avoids causing the sample gas Sg, and dust in the probe tube 11, from coming into contact with the reflection mirror 22. Therefore, the contamination and corrosion of the reflection mirror 22 can be inhibited.

In addition, as shown in Fig. 2, holes 67 and 68 are formed at the both ends of the introducing openings 111, and at the opposite side of the introducing openings 111 (at the side of the upper stream of the sample gas Sg) of the probe tube 11. Flowing the sample gas Sg from these holes 67 and 68 can prevent the purge air Pa from flowing into the middle part of the probe tube 11. The purge air Pa is expelled from the introducing openings 111 (SgPa) while mixing with the sample gas Sg. The introducing openings 111 are also used as an outlet for exhausting the purge air Pa.

The flange 13 is the member that fixes the measurement unit 1 to a funnel 500 that expels the sample gas Sg or to a container that encapsulates the sample gas Sg (See Fig. 2). The flange 13 is, for example, a disk-like member and arranged so as to be passed through by the probe tube 11 at the one end side (the side connected to the optical unit 12) of the probe tube 11. The flange 13 is fixed to the funnel 500 with bolts, for example.

Next, the optical path of the measurement light Lb1 emitted from the light emitting unit 21 will be explained. The propagation path of the measurement light Lb1 is shown in a chain line in Fig. 2. As shown in Fig. 2, the measurement light Lb1 emitted from the light emitting unit 21 passes through the space inside the probe tube 11 and is reflected by the reflection mirror 22. The probe tube 11 is filled with the sample gas Sg. The measurement light that has been reflected by the reflection mirror 22 passes through the space inside the probe tube 11 and propagates toward the light receiving unit 24. Thus, the measurement light Lb1 reciprocates through the space inside the probe tube and is received by the light receiving unit 24.

Here, the measurement light Lb1 that has been reflected by the reflection mirror 22 might be refracted due to so-called thermal lens effect, and may propagate in a path different than straight from the reflection mirror 22 to the light receiving unit 24 in the probe tube 11. In more detail, the sample gas Sg and the purge air Pa flow into the probe tube 11. The spatial temperature gradient might be generated when the temperature difference between the sample gas Sg and the purge gas Pa exists. Thus, the change of the spatial refractive index might be generated in accordance with the spatial temperature gradient and therefore the measurement light Lb1 might be refracted.

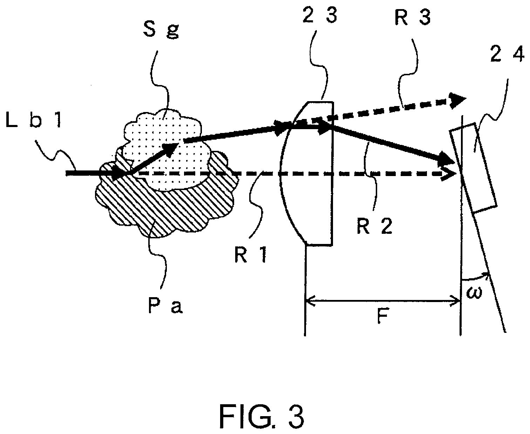

Taking this point into consideration, the measurement unit 1 includes the condensing lens 23. With the measurement unit 1 including the condensing lens 23, the measurement light Lb1 can be guided within the light receiving plane of the light receiving unit 24 by changing the propagation direction of the refracted measurement light Lb1, as shown in Fig. 3. Fig. 3 is an image view showing that the measurement light Lb1 refracted in the measurement unit 1 is guided to the light receiving plane of the light receiving unit 24 by the condensing lens 23. Specifically, as shown in Fig. 3, the measurement light Lb1, which is refracted due to the thermal lens effect, enters the condensing lens 23 and propagates on a path R2. Then, the measurement light Lb1 finally reaches within the light receiving plane of the light receiving unit 24. Without the condensing lens 23, the measurement light Lb1, which is refracted due to the thermal lens effect, propagates on a path R3.

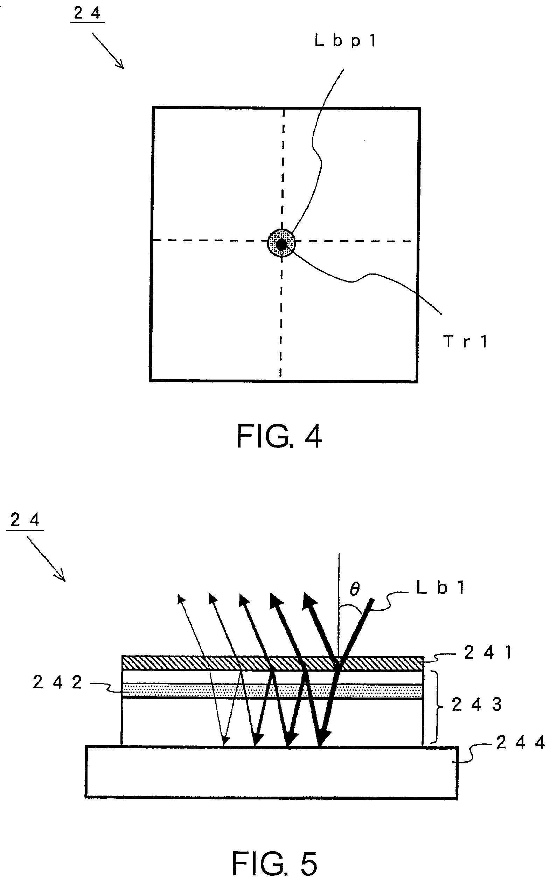

In addition, with the measurement unit 1, the measurement light Lb1 entering the condensing lens 23 is condensed to the predetermined condensing point, in accordance with the property of the condensing lens 23. Therefore, unnecessary movement of the irradiation point Lbp1 can be inhibited. The irradiation point Lbp1 is a point where the measurement light Lb1 intersects the light receiving plane of the light receiving unit 24, as shown in Fig. 4. Fig. 4 is a view showing that the movement of the irradiation point Lbp1 on the light receiving plane is inhibited in the measurement unit 1. In Fig.4, the locus line Tr1 shows the movement locus of the irradiation point Lbp1. Since the locus line Tr1 does not snake in Fig. 4, it is shown that the movement of the irradiation point Lbp1 is inhibited as described above. Thus, with the measurement unit 1 according to the present embodiment, the measurement light Lb1 can be received within the predetermined area of the light receiving plane of the light receiving unit 24. Therefore, a stable light receiving signal can be derived even with the light receiving unit 24 that has a positional dependence of the detection sensitivity.

As described above, with the measurement unit 1, the measurement light Lb1 that has reciprocated inside the probe tube 11 can be properly received within the light receiving plane of the light receiving unit 24. By receiving the measurement light Lb1 within the light receiving plane of the light receiving unit 24, an electrical signal that corresponds to the intensity of the measurement light Lb1 can be derived. Therefore, the gas analyzing apparatus 100, comprising the measurement unit 1, can analyze the sample gas Sg accurately based on the electric signal that corresponds to the intensity of the measurement light Lb1.

In probe-type gas analyzing apparatuses, the proportion of the purge air relative to the sample gas is greater than that in the open-path type apparatus. This is because the sample gas and the purge air are introduced into a limited space inside the probe tube. In other words, the refraction of the measurement light due to the thermal lens effect is greater in a probe-type gas analyzing apparatus than in an open-path type gas analyzing apparatus. Therefore, it is effective to apply the present embodiment to the probe-type measurement unit and the gas analyzing apparatus using the probe-type measurement unit.

According to the invention the numerical aperture NA (Numerical Aperture) of the lens used as the condensing lens 23 is 0.08 or more.

The numerical aperture NA is the value expressed by the equation (1), where $\phi$ is the maximum angle of the light beam, which the condensing lens 23 condenses, relative to the optical axis of the condensing lens 23, n is the refractive index of the medium between the condensing lens 23 and the light receiving unit 24.

$$NA = n\sin\phi \dots (1)$$

Namely, the numerical aperture NA is the value proportional to sine of the condensing angle of the condensing lens 23.

In addition, the light receiving unit 24 is arranged while being tilted relative to the condensing lens so that a tilting angle $\omega$ is 10 greater than or equal to degrees, where the tilting angle $\omega$ is the angle of the light receiving plane of the light receiving unit 24 relative to the image forming plane of the condensing lens 23. Thus, since the multiple reflections can be inhibited without increasing the numerical aperture NA to an extremely large value, the space for setting the distance between the condensing lens 23 and the light receiving unit 24 can be increased. Moreover, it can prevent the incident light from reflecting, returning, and then becoming a noise in the signal.

The reasons why the numerical aperture of the condensing lens 23 is greater than or equal to 0.08, and the tilting angle $\omega$ is greater than or equal to 10 degrees, will explained below.

The light receiving plane of the above-described light receiving unit 24 has multiple layers of semiconductors as shown in Fig. 5. Fig. 5 is a cross-sectional view showing the detailed structure of the light receiving unit 24. Specifically, the light receiving unit 24 includes a package substrate 244, an InP wafer layer 243 arranged on the principal surface of the package substrate 244, an InGaAs absorbing layer 242 formed in the InP wafer layer 243, and an AR (Anti Reflection) coating layer 241 formed on the surface of the InP wafer layer 243. Gold plating is formed on the surface of the package substrate 244. The plane where the AR coating layer 241 is formed is the light receiving plane of the light receiving unit 24. The measurement light Lb1 entering the light receiving surface of the light receiving unit 24 is absorbed by the InGaAs absorbing layer 242. Then, the light receiving unit 24 generates an electric signal in accordance with the intensity of the light absorbed by the InGaAs absorbing layer 242, and outputs this signal to the processing apparatus 30. Several well-known techniques can be used as the technique with which the light receiving unit 24 performs the photoelectric conversion of the measurement light Lb1.

In the conventional techniques, there has been the case in which an electric signal that corresponds to the intensity of the measurement light Lb1 cannot be derived accurately. This is because the multiple reflection of the measurement light Lb1 at the interfaces of the semiconductor layers shown in Fig. 5 causes the interference of the measurement light Lb1 (so-called etalon effect) when the measurement light Lb1 is received by the light receiving unit 24. In more detail, the measurement light Lb1 entering the light receiving unit 24 propagates into the InP wafer layer 243, while a part of the measurement light Lb1 is reflected by the AR coating layer 241. After a part of the measurement light Lb1 is absorbed by the InGaAs absorbing layer 242 in the InP wafer layer 243, the measurement light Lb1 transmits through these layers and is reflected at the surface of the package substrate 244. The measurement light Lb1 that has been reflected at the surface of the package substrate 244 transmits again through the InP wafer layer 243 and the InGaAs absorbing layer 242 and is then reflected again at the interface between the AR coating layer 241 and the InP wafer layer 243. Thus, there has been the case in which, when the measurement light Lb1 enters the light receiving plane of the light receiving unit 24 at the predetermined angle of incidence, the measurement light Lb1 is repeatedly reflected from the interfaces of the semiconductor layers that form the light receiving unit 24. Then the reflected measurement light and the incident measurement light interfere with each other. There has also been the case in which, when such interference occurs, even if the intensity of the measurement light Lb1 is constant at the time when the measurement light Lb1 enters the light receiving unit 24, the magnitude of the electric signal derived from the light receiving unit 24 becomes unstable because the amount of the measurement light Lb1 absorbed in the InGaAs absorbing layer 242 is unstable.

Considering the above, it is preferable that the etalon effect is inhibited in the measurement unit 1. In order to achieve this, the multiple reflections are inhibited by increasing the angle of incidence $\theta$ of the measurement light Lb1 when the measurement light Lb1 enters the light receiving plane of the light receiving unit 24. Here, the angle of incidence $\theta$ can be large as the numerical aperture NA becomes large. In addition, the angle of incidence $\theta$ can also be adjusted by tilting the light receiving plane of the light receiving unit 24 with respect to the optical axis of the condensing lens 23. The inventor considering this point has concluded, by performing the experiments that will be described later, that the numerical aperture NA of the condensing lens 23 is preferably greater than or equal to 0.08 and further the angle of incidence $\theta$ is preferably greater than or equal to 10 degrees.

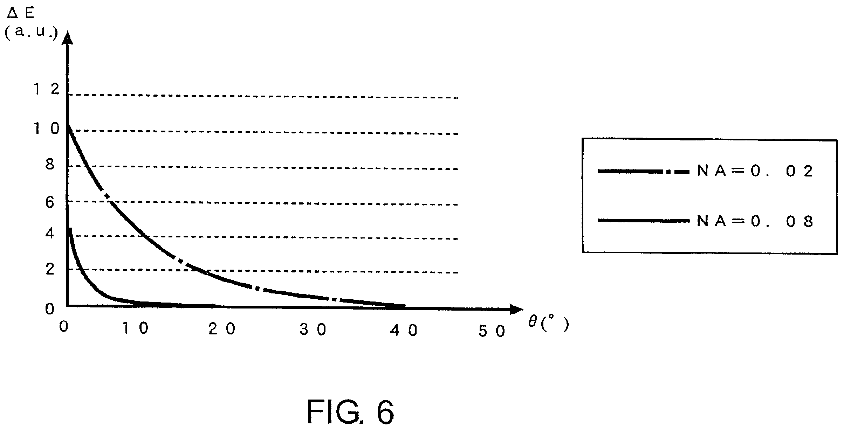

The results of the experiments derived by choosing various values of the numerical apertures NA of the condensing lens and the tilting angles $\omega$ in the measuring unit. 1 will be presented below. Fig. 6 is a graph showing the stabilities of the electrical signals of the light receiving unit 24, derived at each of settings of the condensing lens 23 and the light receiving unit 24 in the measurement unit 1, according to the first embodiment. The vertical axis of Fig. 6 shows the difference value $\Delta E$ (a.u.) between the peak value and the bottom value of the electrical signals of the light receiving unit 24, measured at the corresponding numerical aperture NA and the corresponding angle of incidence $\theta$. The horizontal axis of Fig. 6 shows the angle of incidence $\theta$. In Fig. 6, the chain line shows the difference value $\Delta E$ when the lens with NA value of 0.02 is used as the condensing lens 23 and the solid line shows the difference value $\Delta E$ when the lens with NA value of 0.08 is used as the condensing lens 23.

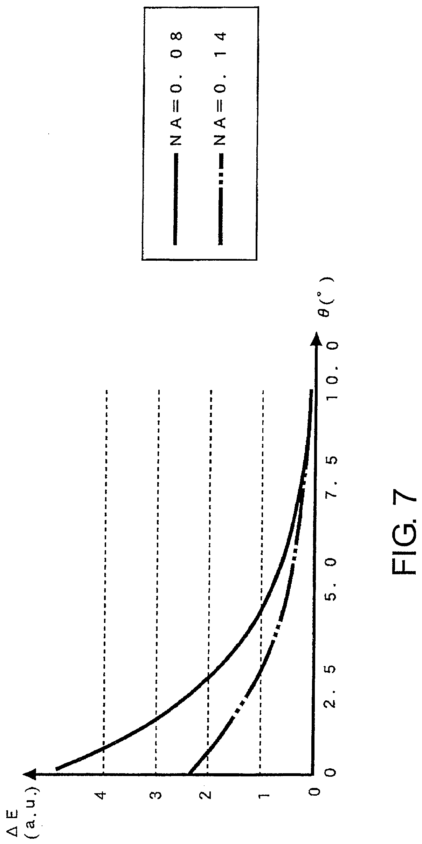

In addition, as shown in Fig. 6 and Fig. 7, it has been found that the difference value $\Delta E$ can be converged to the value extremely close to 0 when the angle of incidence is greater than or equal to 10 degrees when the value of the numerical aperture is greater than or equal to 0.08. Fig. 7 is an enlarged view of a part of Fig. 6. The vertical axis and the horizontal axis of Fig. 7 show the same parameters as those in Fig. 6. In Fig. 7, the solid line shows the difference value $\Delta E$ when the lens with NA value of 0.08 is used as the condensing lens 23 and the chain double-dashed line shows the difference value $\Delta E$ when the lens with NA value of 0.14 is used as the condensing lens 23.

As shown in Fig. 6 and Fig. 7, by setting the numerical aperture NA of the condensing lens 23 to be greater than or equal to 0.08, a more accurate electrical signal can be derived from the light receiving unit 24. Additionally, by setting the angle of incidence $\theta$ to be greater than or equal to 10 degrees, a more accurate electrical signal can be derived. Therefore, in the gas analyzing apparatus 100 comprising the measurement unit 1 in which the condensing lens 23 and the light receiving unit 24 are set in such a manner, the analysis of the sample gas Sg can be performed more accurately based on the more accurate electrical signal.

(Alternative example, which does not form part of the invention) In the above-described first embodiment, the example in which the present invention is applied to the probe-type measurement unit has been shown. An alternative example relates to an open-path measurement unit. The measurement unit 2 according to this example and the gas analyzing apparatus 200 using the measurement unit 2 will be explained below. The elements that are the same as those in the above-described first embodiment are assigned to the same numerals as those in the first embodiment, and the detailed explanations are omitted.

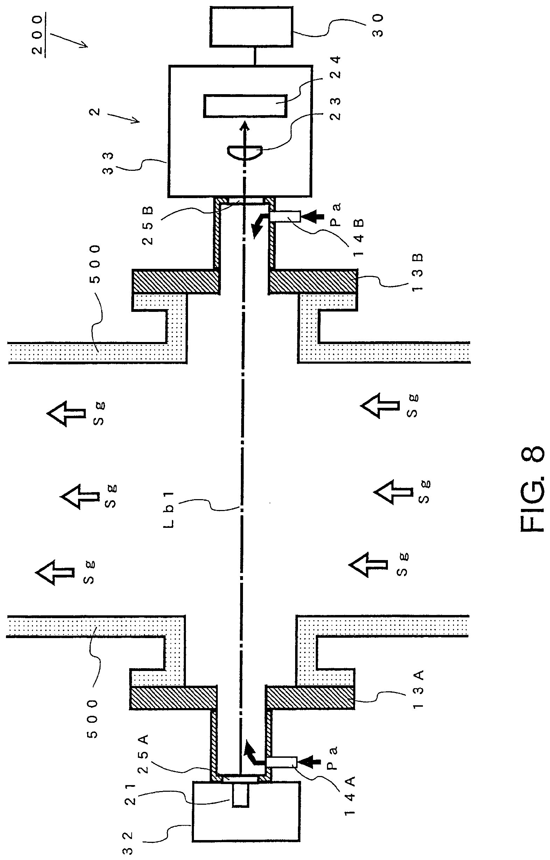

Fig. 8 is a cross-sectional view showing the inner structure of the measurement unit 2. As shown in Fig. 8, the measurement unit 2 includes an oscillator unit 32 and a detector unit 33 that are formed separately. The oscillator unit 32 is attached at one side plane of a funnel 500. The sample gas Sg flows in the funnel 500. The detector unit 33 is attached to a different side plane of the funnel 500, so that the oscillator unit 32 and the detector unit 33 face each other.

The oscillator unit 32 includes a light emitting unit 21, an optical window 25A, a purge air introducing port 14A, and a flange 13A. The optical window 25A is arranged immediately in front of the light emitting unit 21 and the purge air introducing port 14A introduces the purge air Pa into the space that is connected to the funnel 500 immediately in front of the optical window 25A. The detector unit 33 includes a condensing lens 23, a light receiving unit 24, an optical window 25B, a purge air introducing port 14B, and a flange 13B. The condensing lens 23 is arranged immediately in front of the light receiving unit 24. The optical window 25B is arranged immediately in front of the condensing lens 23. The purge air introducing port 14B introduces the purge air Pa into the space that is connected to the funnel 500 immediately in front of the optical window 25B.

The oscillator unit 32 and the detector unit 33 are attached to the funnel 500 via the flanges 13A and 13B, respectively, while their positions are adjusted in advance, so that the measurement light Lb1 emitted by the light emitting unit 21 is emitted toward the light receiving unit 24.

With the above-described measurement unit 2, like the first embodiment, the measurement light Lb1 can be condensed properly within the light receiving plane of the light receiving unit 24 even if the measurement light Lb1 is bent due to the thermal lens effect caused by the purge air Pa and the sample gas Sg. It is also preferable in this example that the numerical aperture NA is set to be greater than or equal to 0.08 and further the angle of incidence $\theta$ is set to be greater than or equal to 10 degrees.

## INDUSTRIAL APPLICABILITY

The measurement unit and the gas analyzing apparatus according to the present invention are useful for the measurement unit, the gas analyzing apparatus, etc. that can analyze the sample gas more accurately than the conventional ones.

## EXPLANATION OF REFERENCE NUMERALS

| | |
|---|---|
| 100, 200 | gas analyzing apparatus |
| 1, 2 | measurement unit |
| 11 | probe tube |
| 12 | optical unit |
| 13 | flange |
| 14 | purge air introducing port |
| 16 | purge air introducing pipe |
| 21 | light emitting unit |
| 22 | reflection mirror |
| 23 | condensing lens |
| 24 | receiving unit |
| 30 | processing apparatus |
| 241 | AR coating layer |
| 242 | InGaAs absorbing layer |
| 243 | InP wafer layer |
| 244 | package substrate |
| 32 | oscillator unit |
| 33 | detector unit |
| 54 | light receiving sensor |

## Claims

1. A measurement unit (1, 2) configured to be used in an analyzing apparatus (100, 200) for measuring concentrations of component gases in a sample gas (Sg) expelled in a funnel (500) of the analyzing apparatus (100, 200), the measurement unit (1) comprising:

at least one flange member (13; 13A, 13B) that is configured to fix the measurement unit (1, 2) to an outside of the funnel (500);
a light emitting unit (21) arranged inside a casing of an optical unit (12) of the measurement unit (1, 2), and configured to emit a measurement light (Lbl) to the sample gas along an optical path extending to the inside of the funnel (500), the light emitting unit (21) being a laser oscillating apparatus or a LED;
a light receiving unit (24) arranged in the casing of the optical unit (12), and configured to receive the measurement light on a light receiving plane formed with a multi-layered semiconductor;
a purge air introducing unit (14; 16) configured to introduce a purge air (Pa) into a tubular portion of the measurement unit, the tubular portion being arranged outside of the funnel (500) between the flange member (13; 13A, 13B) and the light emitting unit (21), and being in fluid connection with the inside of the funnel (500);
a cylindrical probe tube (11) having openings (67, 68, 111) to introduce the sample gas into the probe tube (11), wherein the purge air introducing unit (14; 16) is further configured to introduce the purge air (Pa) inside the probe tube (11), and the light emitting unit (21) is configured to emit the measurement light (Lbl) to the sample gas introduced inside the probe tube (11);
a condensing lens (23) arranged in the optical path and inside the casing of the optical unit (12), and configured to condense the measurement light (Lbl) within the light receiving plane of the light receiving unit (24), the optical path being a path of the measurement light (Lbl) extending from the light emitting unit (21) to the light receiving unit (24), a propagation path of the measurement light (Lbl) within the probe tube (11) being subject to a thermal lens effect caused in use of the measurement unit in an analyzing apparatus (100, 200) by a temperature difference between the sample gas (Sg) and the purge air (Pa) being introduced by the purge air introducing unit (14; 16) in the tubular portion and the probe tube (11); and
at least one optical window (25, 25A, 25B) arranged at the casing of the optical unit (12), the optical window (25, 25A, 25B) being disposed in front of the light emitting unit (21) and the condensing lens (23) and configured to protect the light emitting unit (21) and the condensing lens (23),
wherein the purge air introducing unit (14; 16) is configured to introduce the purge air (Pa) into the tubular portion at the side of the optical window (25, 25A, 25B) opposite to the side where the light emitting unit (21) and the condensing lens (23) are arranged,

wherein a numerical aperture of the condensing lens (23) is greater than or equal to 0.08, and wherein the light receiving unit (24) is tilted with respect to the condensing lens (23) such that an angle between the light receiving plane and an image formation plane of the condensing lens (23) is greater than or equal to 10 degrees.

2. The measurement unit according to claim 1, wherein the condensing lens (23) is arranged immediately in front of the light receiving unit (24), and the optical window (25) is arranged immediately in front of the condensing lens (23).

3. The measurement unit according to claim 1 or 2, further comprising a reflection mirror (22) arranged at one end portion of the probe tube (11), wherein

the light emitting unit (21) is arranged at another end portion of the probe tube (11), the light emitting unit (21) being configured to emit the measurement light (Lbl) toward the reflection mirror (22), and
the light receiving unit (24) is arranged at the another end portion of the probe tube (11), the light receiving unit (24) being configured to receive the measurement light (Lbl) that has been reflected by the reflection mirror (22).

4. A gas analyzing apparatus (100, 200) comprising:

a measurement unit (1, 2) according to any one of claims 1 to 3; and
a processing apparatus configured to calculate concentrations of component gases in a sample gas based on a signal received from the light receiving unit (24).

**Patentansprüche**

1. Messeinheit (1, 2), die dafür konfiguriert ist, in einer Analysiervorrichtung (100, 200) zum Messen von Konzentrationen von Komponentengasen in einem Probengas (Sg), das in einem Schacht (500) der Analysiervorrichtung (100, 200) ausgestoßen wird, verwendet zu werden, wobei die Messeinheit (1) aufweist:

zumindest ein Flanschelement (13; 13A, 13B), das dafür konfiguriert ist, die Messeinheit (1, 2) an einer Außenseite des Schachts (500) zu befestigen;
eine lichtemittierende Einheit (21), die im Innern eines Gehäuses einer optischen Einheit (12) der Messeinheit (1, 2) angeordnet und dafür konfiguriert ist, ein Messlicht (Lb1) zum Probengas entlang einem optischen Weg zu emittieren, der

in das Innere des Schachts (500) verläuft, wobei die lichtemittierende Einheit (21) eine Laser-Oszillationsvorrichtung oder eine LED ist;
eine lichtempfangende Einheit (24), die im Gehäuse der optischen Einheit (12) angeordnet und dafür konfiguriert ist, das Messlicht auf einer lichtempfangenden Ebene, die mit einem mehrschichtigen Halbleiter ausgebildet ist, zu empfangen;
eine Spülluft einführende Einheit (14; 16), die dafür konfiguriert ist, eine Spülluft (Pa) in einen rohrförmigen Teil der Messeinheit einzuführen, wobei der rohrförmige Teil außerhalb des Schachts (500) zwischen dem Flanschelement (13; 13A, 13B) und der lichtemittierenden Einheit (21) angeordnet ist und mit dem Inneren des Schachts (500) in Fluidverbindung steht;
ein zylindrisches Sondenrohr (11) mit Öffnungen (67, 68, 111), um das Probengas in das Sondenrohr (11) einzuführen, wobei die Spülluft einführende Einheit (14; 16) ferner dafür konfiguriert ist, die Spülluft (Pa) in das Innere des Sondenrohrs (11) einzuführen, und die lichtemittierende Einheit (21) dafür konfiguriert ist, das Messlicht (Lb1) zu dem in das Innere des Sondenrohrs (11) eingeführten Probengas zu emittieren;
eine Kondensorlinse (23), die im optischen Weg und im Innern des Gehäuses der optischen Einheit (12) angeordnet und dafür konfiguriert ist, das Messlicht (Lb1) innerhalb der lichtempfangenden Ebene der lichtempfangenden Einheit (24) zu sammeln, wobei der optische Weg ein Weg des Messlichts (Lb1) ist, der von der lichtemittierenden Einheit (21) zur lichtempfangenden Einheit (24) verläuft, wobei ein Ausbreitungsweg des Messlichts (Lb1) innerhalb des Sondenrohrs (11) einem thermischen Linseneffekt ausgesetzt ist, der bei Verwendung der Messeinheit in einer Analysiervorrichtung (100, 200) durch eine Temperaturdifferenz zwischen dem Probengas (Sg) und der Spülluft (Pa), die durch die Spülluft einführende Einheit (14; 16) in den rohrförmigen Teil und das Sondenrohr (11) eingeführt wird, hervorgerufen wird; und
zumindest ein optisches Fenster (25, 25A, 25B), das am Gehäuse der optischen Einheit (12) angeordnet ist, wobei das optische Fenster (25, 25A, 25B) vor der lichtemittierenden Einheit (21) und der Kondensorlinse (23) angeordnet und dafür konfiguriert ist, die lichtemittierende Einheit (21) und die Kondensorlinse (23) zu schützen,
wobei die Spülluft einführende Einheit (14; 16) dafür konfiguriert ist, die Spülluft (Pa) in den rohrförmigen Teil an der Seite des optischen Fensters (25, 25A, 25B) einzuführen, die der Seite entgegengesetzt ist, wo die lichtemittierende Einheit (21) und die Kondensorlinse (23) angeordnet sind,

wobei eine numerische Apertur der Kondensorlinse (23) größer als oder gleich 0,08 ist, und wobei die lichtempfangende Einheit (24) in Bezug auf die Kondensorlinse (23) so geneigt ist, dass ein Winkel zwischen der lichtempfangenden Ebene und einer Bilderzeugungsebene der Kondensorlinse (23) größer als oder gleich 10 Grad ist.

2. Messeinheit nach Anspruch 1, wobei die Kondensorlinse (23) unmittelbar vor der lichtempfangenden Einheit (24) angeordnet ist und das optische Fenster (25) unmittelbar vor der Kondensorlinse (23) angeordnet ist.

3. Messeinheit nach Anspruch 1 oder 2, ferner aufweisend einen Reflexionsspiegel (22), der an einem Endteil des Sondenrohrs (11) angeordnet ist, wobei

die lichtemittierende Einheit (21) an einem anderen Endteil des Sondenrohrs (11) angeordnet ist, wobei die lichtemittierende Einheit (21) dafür konfiguriert ist, das Messlicht (Lb1) in Richtung des Reflexionsspiegels (22) zu emittieren, und die lichtempfangende Einheit (24) am anderen Endteil des Sondenrohrs (11) angeordnet ist, wobei die lichtempfangende Einheit (24) dafür konfiguriert ist, das Messlicht (Lb1), das vom Reflexionsspiegel (22) reflektiert wurde, zu empfangen.

4. Gasanalysiervorrichtung (100, 200), aufweisend:

eine Messeinheit (1, 2) nach einem der Ansprüche 1 bis 3; und eine Verarbeitungsvorrichtung, die dafür konfiguriert ist, Konzentrationen von Komponentengasen in einem Probengas basierend auf einem von der lichtempfangenden Einheit (24) empfangenen Signal zu berechnen.

**Revendications**

1. Unité de mesure (1, 2) configurée pour être utilisée dans un dispositif d'analyse (100, 200) pour mesurer des concentrations de composants gazeux dans un gaz échantillon (Sg) expulsé dans un entonnoir (500) du dispositif d'analyse (100, 200), l'unité de mesure (1) comprenant :

au moins un organe de bride (13 ; 13A, 13B) qui est configuré pour fixer l'unité de mesure (1, 2) à un extérieur de l'entonnoir (500) ; une unité émettrice de lumière (21) agencée dans un boîtier d'une unité optique (12) de l'unité de mesure (1, 2) et configurée pour émettre une lumière de mesure (Lbl) vers le gaz échantillon, le long d'un chemin optique s'étendant vers l'intérieur de l'entonnoir (500), l'unité émettrice de lumière (21) étant un dispositif d'oscillation de laser ou une DEL ;

une unité réceptrice de lumière (24) agencée dans le boîtier de l'unité optique (12), et configurée pour recevoir la lumière de mesure sur un plan de réception de lumière formé avec un semi-conducteur multicouche ;

une unité d'introduction d'air de purge (14 ; 16) configurée pour introduire un air de purge (Pa) dans une partie tubulaire de l'unité de mesure, la partie tubulaire étant agencée à l'extérieur de l'entonnoir (500), entre l'organe de bride (13 ; 13A, 13B) et l'unité émettrice de lumière (21), et étant en communication fluidique avec l'intérieur de l'entonnoir (500) ;

un tube de sonde cylindrique (11) ayant des ouvertures (67, 68, 111) pour introduire le gaz échantillon dans le tube de sonde (11), dans laquelle l'unité d'introduction d'air de purge (14 ; 16) est en outre configurée pour introduire l'air de purge (Pa) dans le tube de sonde (11) et l'unité émettrice de lumière (21) est configurée pour émettre la lumière de mesure (Lbl) vers le gaz échantillon introduit dans le tube de sonde (11) ;

une lentille condensatrice (23) agencée sur le chemin optique et à l'intérieur du boîtier de l'unité optique (12) et configurée pour condenser la lumière de mesure (Lbl) dans le plan de réception de lumière de l'unité réceptrice de lumière (24), le chemin optique étant un chemin de la lumière de mesure (Lb1) s'étendant de l'unité émettrice de lumière (21) à l'unité réceptrice de lumière (24), un chemin de propagation de la lumière de mesure (Lbl) dans le tube de sonde (11) étant soumis à un effet thermique de lentille provoqué lors de l'utilisation de l'unité de mesure dans un dispositif d'analyse (100, 200) par une différence de température entre le gaz échantillon (Sg) et l'air de purge (Pa) étant introduit par l'unité d'introduction d'air de purge (14 ; 16) dans la partie tubulaire et le tube de sonde (11) ; et

au moins une fenêtre optique (25, 25A, 25B) agencée en le boîtier de l'unité optique (12), la fenêtre optique (25, 25A, 25B) étant disposée devant l'unité émettrice de lumière (21) et la lentille condensatrice (23) et configurée pour protéger l'unité émettrice de lumière (21) et la lentille condensatrice (23),

dans laquelle l'unité d'introduction d'air de purge (14 ; 16) est configurée pour introduire l'air de purge (Pa) dans la partie tubulaire, du côté de la fenêtre optique (25, 25A, 25B) opposé au côté où sont agencées l'unité émettrice de lumière (21) et la lentille condensatrice (23),

dans laquelle une ouverture numérique de la lentille condensatrice (23) est supérieure ou égale à 0,08, et

dans laquelle l'unité réceptrice de lumière (24) est inclinée par rapport à la lentille condensatrice (23) de sorte qu'un angle entre le plan de réception de lumière et un plan de formation d'image de la lentille condensatrice (23) soit supérieur ou égal à 10 degrés.

2. Unité de mesure selon la revendication 1, dans lequel la lentille condensatrice (23) est agencée immédiatement devant l'unité réceptrice de lumière (24), et la fenêtre optique (25) est agencée immédiatement devant la lentille condensatrice (23).

3. Unité de mesure selon la revendication 1 ou 2, comprenant en outre un miroir réfléchissant (22) agencé en une partie terminale du tube de sonde (11), dans laquelle

l'unité émettrice de lumière (21) est agencée en une autre partie terminale du tube de sonde (11), l'unité émettrice de lumière (21) étant configurée pour émettre la lumière de mesure (Lb1) vers le miroir réfléchissant (22), et

l'unité réceptrice de lumière (24) est agencée en l'autre partie terminale du tube de sonde (11), l'unité réceptrice de lumière (24) étant configurée pour recevoir la lumière de mesure (Lb1) qui a été réfléchie par le miroir réfléchissant (22).

4. Dispositif d'analyse de gaz (100, 200) comprenant :

une unité de mesure (1, 2) selon l'une des revendications 1 à 3 ; et

un dispositif de traitement configuré pour calculer des concentrations de composants gazeux d'un gaz échantillon sur la base d'un signal reçu de l'unité réceptrice de lumière (24).

FIG. 1

FIG. 2

FIG. 3

2 4

L b p 1

T r 1

FIG. 4

2 4

$\theta$   L b 1

2 4 1

2 4 2

2 4 3

2 4 4

FIG. 5

FIG. 6

FIG. 7

FIG. 8

S g 2    R 3

L b 2

R 1

P a 2

5 4

FIG. 9

5 4

L b p 2

T r 2

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6809825 B
- US 6044329 A
- US 4549080 A
- US 2006176486 A
- US 5984998 A
- US 4560873 A
- US 4126396 A